(19) Europäisches Patentamt
European Patent Office
Office européen des brevets

(11) Numéro de publication: **0 232 666 B1**

# (12) FASCICULE DE BREVET EUROPEEN

(45) Date de publication du fascicule du brevet: **08.11.89**

(51) Int. Cl.⁴: **C 07 C 53/48**, C 07 C 51/58

(21) Numéro de dépôt: **86420309.6**

(22) Date de dépôt: **24.12.86**

(54) **Procédé de préparation de chlorure de trifluoroacétyle.**

(30) Priorité: **31.12.85 FR 8519567**

(43) Date de publication de la demande: **19.08.87 Bulletin 87/34**

(45) Mention de la délivrance du brevet: **08.11.89 Bulletin 89/45**

(84) Etats contractants désignés: **AT BE CH DE ES FR GB GR IT LI LU NL SE**

(56) Documents cités: **DE-A-1 285 999**

(73) Titulaire: **RHONE-POULENC CHIMIE, 25, quai Paul Doumer, F-92408 Courbevoie Cédex (FR)**

(72) Inventeur: **Correia, Yves, "Les Lauzières", F-04160 - Chateau-Arnoux (FR)**
Inventeur: **Vermont, Jean, 9, rue du Languedoc, F-04600 - Saint-Auban Sur Durance (FR)**

(74) Mandataire: **Le Pennec, Magali et al, RHONE-POULENC INTERSERVICES Service Brevets Chimie 25, Quai Paul Doumer, F-92408 Courbevoie Cédex (FR)**

EP 0 232 666 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description**

La présente invention concerne un nouveau procédé de préparation du chlorure de trifluoroacétyle. Elle concerne plus particulièrement un procédé de préparation du chlorure de trifluoroacétyle à partir des esters du trifluoroéthanol.

Les procédés actuellement connus de préparation du chlorure de trifluoroacétyle peuvent être divisés en 3 groupes:

Le premier groupe consiste à chlorer l'acide trifluoroacétique ou un de ses dérivés: anhydride ou sel de sodium par divers agents de chloration: chlorure de sulfonyle, pentachlorure de phosphore [Am. Soc. 71 (1949) 752], phénylchloroforme, acide chlorobenzoïque [Am. Soc. 70 (1948) 1968], tétra chlorure de silicium.

Le deuxième groupe consiste à effectuer une chloration photochimique du trifluoroacétaldéhyde, plus connu sous la dénomination de fluoral, en l'absence ou en présence de charbon actif (demande de brevet européen n° 108 675).

Le troisième groupe consiste à effectuer une oxydation du perhalogénoéthane (Journal of Chemical Society, 1959, 387-96) ou de perhalogénobutène (brevet hollandais n° 6 611 128).

Aucun des documents précédents n'utilise comme matière première pouvant subir une chloration le trifluoroéthanol ou un de ses esters. Les esters formés à partir du trifluoroéthanol et qui répondent à la formule générale (I) suivante:

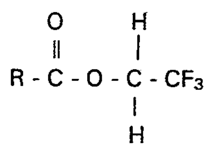

(I)

dans laquelle R représente le chlore, le fluor, un groupe perhalogénoalkyle de formule - $C_n Cl_x F_y$ dans laquelle x + y est égal à 2n + 1 et n est compris entre 1 et 4 peuvent être utilisés comme matière première dans le procédé de la présente invention qui consiste à effectuer une chloration desdits esters et à effectuer simultanément ou successivement une mise en contact avec une amine tertiaire ou un acide de Lewis.

Parmi les esters de formule (I) on peut préparer par exemple: le chloroformiate de trifluoroéthyle selon l'enseignement du brevet US 3 852 464 exemple 1 par contact de trifluoroéthanol avec le phosgène; le trifluoroacétate de trifluoroéthyle selon l'article de Bournes, Stacey, Tatlow, Worral paru dans le Journal of Chemical Society, 1958, 3268-82 par estérification de l'anhydride trifluoroacétique par l'alcool trifluoroéthylique ou selon les méthodes classiques d'estérification de l'acide trifluoroacétique par l'alcool trifluoroéthylique.

Parmi les esters de formule (I) on préfère utiliser ceux par lesquels R est un radical perhaloalkyle comprenant un atome de carbone et tout particulièrement le trichloroacétate de trifluoroéthyle ou le trifluoroacétate de trifluoroéthyle.

La chloration est effectuée en présence de chlore et d'un système capable de fournir du chlore radicalaire tel que par exemple: la lumière, les initiateurs de radicaux libres notamment l'azobisisobutyronitrile et le peroxyde de benzoyle.

On peut citer comme amines tertiaires utilisables dans le procédé de la présente invention: la triéthylamine, la tributylamine. Parmi les amines tertiaires ou les chlorhydrates d'amines tertiaires on préfère utiliser la triéthylamine ou son chlorhydrate. On peut citer parmi les acides de Lewis, le chlorure ferrique, le chlorure d'aluminium. La chloration peut s'effectuer en présence ou en l'absence de solvant. Lorsqu'elle s'effectue en présence de solvant, celuici doit être inerte vis à vis du chlore. On préfère ainsi utiliser le tétrachlorure de carbone ou le chlorure de trichloracétyle.

La chloration peut s'effectuer en présence de l'amine mais il y aura alors dégagement simultané au cours de la réaction d'acide chlorhydrique, de chlorure de trifluoroacétyle et du chlore en excès ce qui est valable si on utilise ce mélange gazeux in situ, si non il est préférable d'effectuer la chloration, d'éliminer le chlore en excès et l'acide chlorhydrique formé puis d'ajouter l'amine afin de libérer le chlorure de trifluoroacétyle pur selon les deux réactions chimiques suivantes:

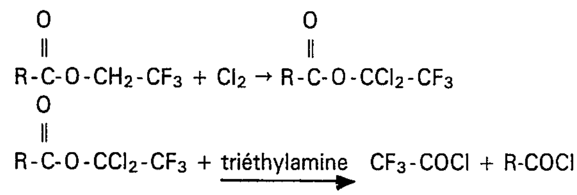

Le chlorure d'acide de formule R COCl, sous produit de la réaction peut être utilisé pour former l'ester par condensation avec le trifluoroéthanol et ainsi recyclé à la première étape d'un procédé plus général qui consiste à former un ester de trifluoroéthane par condensation de trifluoroéthanol avec un acide de formule R COCl en présence d'une amine tertiaire ou d'un acide de Lewis, à chlorer cet ester par le chlore gazeux en présence d'un système capable de fournir du chlore radicalaire et à obtenir le chlorure de trifluoroacétyle par contact avec une amine tertiaire ou un acide de Lewis. Ces trois étapes réactionnelles peuvent être réalisées simultanément dans le milieu réactionnel à condition de conserver l'amine tertiaire ou l'acide de Lewis et de séparer le chlorure de trifluoroacétyle, le chlore et l'acide chlorhydrique issus de la réaction.

Pour une meilleure mise en oeuvre de la présente invention on utilise de préférence un excès de chlore par rapport à la stoechiométrie, cet excès peut être très large et même dépasser 300%.

Lorsque l'on ajoute un initiateur de radicaux libres on l'ajoute de préférence selon un rapport pondéral par rapport à l'ester compris entre 0,1 et 1%.

La réaction de chloration est effectuée de préférence à une température comprise entre 50 et 200°C et à une pression égale ou supérieure à la pression atmosphérique.

Le chlorure de trifluoroacétyle issu du présent procédé peut être utilisé comme catalyseur ou comme intermédiaire de synthèse dans l'industrie pharmaceutique ou phytosanitaire (brevet japonais 58 159 440).

La présente invention va être plus complètement décrite à l'aide des exemples suivantes qui ne doivent en aucun cas être considérés comme limitatifs de l'invention.

*Exemple 1*

Dans un réacteur on maintient à 73°C 1,64 mole (299 g) de chlorure de trichloroacétyle, 0,015 mole (2 g) d'$AlCl_3$ et 1,64 mole (164 g) d'alcool trifluoroéthylique pendant 6 heures puis à 120°C pendant 7 heures supplémentaires.

On distille 1,45 mole (356 g) de trichloracétate de trifluoroéthyle (Eb = 78°C, spectre infra rouge correct, rendement = 88,5%).

On soumet alors à la chloration photochimique (réaction effectuée en présence d'une lampe à vapeur de mercure haute pression) 0,95 mole (233 g) de l'ester préparé ci-dessus dissous dans 400 $cm^3$ de $CCl_4$ maintenu à 60°C. On introduit pendant 4 heures 0,5 mole/h de chlore. On distille 0,865 mole (272 g) de trichloroacétate de dichlorotrifluoroéthyle ($Eb^{13}$ = 57°C, rendement = 91%, spectre infrarouge correct, pas de CH).

On maintient alors à 60°C pendant 1 heure puis 15 mn à 100°C 0,189 mole (59,5 g) de l'ester perhalogéné ci-dessus et 0,002 mole (0,3 g) de chlorhydrate de triéthylamine en piègeant les évents à −70°C. On récupère ainsi 0,166 mole (22 g) de chlorure de trifluoroacétyle (rendement = 87,9%, spectre infra rouge correct pour les fonctions) et 32 g (0,175 mole) 93% de chlorure de trichloracétyle.

*Exemple 2*

Dans un mélange réactionnel maintenu à 115°C et contenant 2 moles (364 g) de chlorure de trichloracétyle et 0,0015 mole (0,2 g) de chlorhydrate de triéthylamine, on introduit en 8 heures 2 moles (200 g) d'alcool trifluoroéthylique.

L'acide chlorhydrique correspondant à la réaction d'estérification est dégagé.

Ce mélange réactionnel est alors photochloré en introduisant un débit de chlore de 0,60 mole/h pendant 9 heures à 100°C en piègeant les évents à −70°C. A la fin de la réaction, on maintient le piège à −30°C pour permettre au chlore de se vaporiser.

On récupère ainsi 1,8 mole (238,5 g) de chlorure de trifluoroacétyle (spectre infra rouge correct, rendement = 90%).

*Exemple 3a*

On procède comme dans l'exemple 2, mais la chloration est réalisée en présence d'azobisisobutyronitrile, 3 ajouts de 1 g au cours des 9 heures et on obtient en traitant comme dans l'exemple 2, 1,7 mole de chlorure de trifluoroacétyle.

*Exemple 3b*

Dans un mélange réactionnel maintenu à 115°C et contenant 2,09 mole (381 g) de chlorure de trichloroacétyle et 0,0015 mole (0,2 g) de chlorhydrate de triéthylamine, on introduit en 8,5 heures 2,09 mole (209 g) d'alcool trifluoroéthylique.

On distille 1,98 mole (485 g) de trichloroacétate de trifluoroéthyle ($Eb^{49}$ = 68°C, spectre infra rouge correct, rendement = 94,4%).

On soumet alors à la chloration photochimique 1,95 mole (478,5 g) de l'ester trifluoroacétique préparé ci-dessus maintenu à 60°C. On introduit pendant 9 heures 0,55 mole/h de chlore.

Quand la chloration est terminée (plus de libération $Cl^-$) on laisse refroidir et on additionne 0,02 mole (0,3 g) de chlorhydrate de triéthylamine. On maintient à 60°C pendant 1 h puis à 100°C pendant 30 mn en piègeant les évents à −70°C.

On récupère ainsi 1,82 mole (282 g) de chlorure de trifluoroacétyle (rendement = 93,3%) et une quantité importante de chlorure de trichloracétyle qui reste dans le bouilleur.

*Exemple 4*

On soumet à la chloration photochimique 1 mole (162,5 g) de chloroformiate de trifluoroéthyle dissous dans 400 $cm^3$ de $CCl_4$ maintenu à 60°C. On introduit pendant 4 heures 0,55 mole/h de chlore.

La masse réactionnelle est balayée à l'azote puis on y ajoute à 0°C 0,3 g de chlorhydrate de triéthylamine sous agitation. On constate un dégagement de gaz constitué d'un mélange de phosgène et de chlorure de trifluoroacétyle (identification infrarouge et en chromatographie en phase gazeuse).

*Exemple 5*

Dans une réacteur de photochloration, on place 196 g (1 mole) de trifluoroacétate de trifluoroéthyle et à 50°C on introduit en 4 heures, 2,5 moles de chlore. On ajoute alors au produit, après élimination du chlore par un entraînement à l'azote, 3 g de chlorhydrate de triéthylamine à une température de 20°C. On observe un dégagement gazeux qui augmente avec l'accroissement de la température du ballon que l'on chauffe.

On recueille, dans un piège à froid maintenu à −70°C, 220 g de chlorure de trifluoroacétyle. Rendement 83%.

*Exemple 6*

On soumet à la chloration photochimique 100 g (1 mole) de trifluoroéthanol en présence de 1 g de chlorhydrate de triéthylamine dans 350 $cm^3$ de tétrachlorure de carbone maintenu à 30°C. Débit de chlore 0,5 mole/heure.

On constate un dégagement de chlorure de trifluoroacétyle avec l'acide chlorhydrique et l'excès de chlore. Celui-ci peut être piégé à −70°C ou utilisé directement pour des synthèses.

**Revendications**

1. Procédé de préparation de chlorure de trifluoroacétyle caractérisé en ce qu'on effectue une chloration d'un ester de formule générale (I)

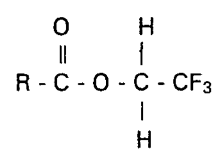

(I)

dans laquelle R représente le chlore, le fluor, un

groupe perhalogénoalkyle contenant 1 à 4 atomes de carbone et en ce que l'on effectue simultanément ou successivement une mise en contact avec une amine tertiaire ou un acide de Lewis.

2. Procédé selon la revendication 1, caractérisé en ce que dans la formule (I) R représente un radical $-C_n F_x Cl_y$ dans lequel x + y est égal à 2n + 1.

3. Procédé selon la revendication 2, caractérisé en ce que l'ester de formule (I) est le trichloracétate de trifluoroéthyle ou le trifluoroacétate de trifluoroéthyle.

4. Procédé sélon la revendication 1, caractérisé en ce que l'amine tertiaire est la triéthylamine.

5. Procédé selon la revendication 1, caractérisé en ce qu'on effectue la chloration de l'ester de formule générale (I) puis la mise en contact avec l'amine.

6. Procédé selon la revendication 1, caractérisé en ce que la chloration est effectuée en présence de la lumière ou d'initiateurs de radicaux libre choisis parmi l'azobisisobutyronitrile et le peroxyde de benzoyle.

7. Procédé selon la revendication 1, caractérisé en ce que la chloration est effectuée en présence d'un excès stoéchiométrique de chlore.

8. Procédé selon la revendication 5, caractérisé en ce que l'initiateur de radicaux libres est ajouté selon un rapport pondéral par rapport à l'ester compris entre 0,1 et 1%.

9. Procédé selon la revendication 1, caractérisé en ce que la chloration est effectuée à une température comprise entre 50 et 200°C.

10. Procédé selon la revendication 1, caractérisé en ce que la chloration est effectuée à la pression atmosphérique ou à une pression supérieure.

**Patentansprüche**

1. Verfahren zur Herstellung von Trifluoracetylchlorid, dadurch gekennzeichnet, daß man einen Ester der allgemeinen Formel (I)

```
    O   H
    ‖   |
R - C - O - C - CF3        (I)
            |
            H
```

in der R Chlor, Fluor, eine Perhalogenalkylgruppe mit 1 bis 4 Kohlenstoffatomen ist, chloriert und gleichzeitig oder anschließend mit einem tertiären Amin oder einer Lewis-Säure in Berührung bringt.

2. Verfahren nach Anspruch 1, dadurch gekennzeichnet, daß R in der Formel (I) der Gruppe $C_n F_x Cl_y$ entspricht, in welcher x + y = 2n + 1 ist.

3. Verfahren nach Anspruch 2, dadurch gekennzeichnet, daß der Ester der Formel (I) Trifluorethyltrichloracetat oder Trifluorethyltrifluoracetat ist.

4. Verfahren nach Anspruch 1, dadurch gekennzeichnet, daß das tertiäre Amin Triethylamin ist.

5. Verfahren nach Anspruch 1, dadurch gekennzeichnet, daß man die Chlorierung des Esters der allgemeinen Formel (I) vornimmt und dann die Berührung mit dem Amin herstellt.

6. Verfahren nach Anspruch 1, dadurch gekennzeichnet, daß man die Chlorierung in Gegenwart von Licht oder von freie Radikale bildenden Initiatoren, ausgewählt unter Azobisisobutyronitril und Benzoylperoxid, vornimmt.

7. Verfahren nach Anspruch 1, dadurch gekennzeichnet, daß man die Chlorierung mit einem Überschuß an Chlor durchführt.

8. Verfahren nach Anspruch 5, dadurch gekennzeichnet, daß man den freie Radikale liefernden Initiator in einer Menge von 0,1 bis 1 Gew.-% – bezogen auf den Ester – verwendet.

9. Verfahren nach Anspruch 1, dadurch gekennzeichnet, daß man de Chlorierung bei einer Temperatur von 50 bis 200°C durchführt.

10. Verfahren nach Anspruch 1, dadurch gekennzeichnet, daß man die Chlorierung bei Atmosphärendruck oder Überdruck durchführt.

**Claims**

1. Process for preparing trifluoroacetyl chloride, characterized in that chlorination of an ester of general formula (I)

```
    O   H
    ‖   |
R - C - O - C - CF3        (I)
            |
            H
```

in which R denotes chlorine, fluorine or a perhaloalkyl group containing 1 to 4 carbon atoms, is performed, and in that a tertiary amine or a Lewis acid is simultaneously or successively introduced.

2. Process according to claim 1, characterized in that, in the formula (I), R denotes a radical $-C_n F_x Cl_y$ in which x + y is equal to 2n + 1.

3. Process according to claim 2, characterized in that the ester of formula (I) is trifluoroethyl trichloroacetate or trifluoroethyl trifluoroacetate.

4. Process according to claim 1, characterized in that the tertiary amine is triethylamine.

5. Process according to claim 1, characterized in that the chlorination of the ester of general formula (I) is performed, followed by the introduction of the amine.

6. Process according to claim 1, characterized in that the chlorination is performed in the presence of light or of free-radical initiators chosen from azobisisobutyronitrile and benzoyl peroxide.

7. Process according to claim 1, characterized in that the chlorination is performed in the presence of a stoichiometric excess of chlorine.

8. Process according to claim 5, characterized in that the free-radical initiator is added in a ratio relative to the ester of between 0.1 and 1% by weight.

9. Process according to claim 1, characterized in that the chlorination is performed at a temperature of between 50 and 200°C.

10. Process according to claim 1, characterized in that the chlorination is performed at atmospheric pressure or at a higher pressure.